# EUROPEAN PATENT SPECIFICATION

(11) **EP 3 536 800 B1**
(45) Date of publication and mention of the grant of the patent: **04.08.2021**
(21) Application number: 18161079.1
(22) Date of filing: 09.03.2018
(51) Int. Cl.: C12P 7/64

(54) **EXTRACTION OF RENEWABLE TRIGLYCERIDES FROM OLEAGINOUS MICROORGANISMS**
EXTRAKTION VON ERNEUERBAREN TRIGLYCERIDEN AUS ÖLHALTIGEN MIKROORGANISMEN
EXTRACTION DE TRIGLYCÉRIDES RENOUVELABLES À PARTIR DE MICRO-ORGANISMES OLÉAGINEUX

(43) Date of publication of application: 11.09.2019
(73) Proprietor: TU München, 80333 München (DE)
(72) Inventor: SOHLING, Ulrich, 85356 Freising (DE); STEGE, Andrea, 85368 Moosburg (DE); SCHIRRMACHER, Georg, 85521 Ottobrunn (DE); MASRI, Mahmoud, 85354 Freising (DE); BRUECK, Thomas, 85452 Eichenried (DE)
(74) Representative: Engelhard, Markus

(56) References cited:
- YELLAPU ET AL: "Recent developments of downstream processing for microbial lipids and conversion to biodiesel", BIORESOURCE TECHNOLOGY, vol. 256, 1 February 2018 (2018-02-01), pages 515-528, XP085361865,
- ZHANG ET AL: "Optimization of enzymatic hydrolysis for effective lipid extraction from microalgae Scenedesmus sp.", RENEWABLE ENERGY, vol. 125, 30 January 2018 (2018-01-30), pages 1049-1057, XP085394396,
- MASRI ET AL: "A seagrass-based biorefinery for generation of single-cell oils for biofuel and oleochemical production", ENERGY TECHNOLOGY, vol. 6, 18 December 2017 (2017-12-18), pages 1026-1038, XP002783301,
- ZHENG ET AL: "Polymer-enhanced enzymatic microalgal cell disruption for lipid and sugar recovery", ALGAL RESEARCH, vol. 14, 2016, pages 100-108, XP002783317,
- ZUORRO ET AL: "Optimization of enzyme-assisted lipid extraction from Nannochloropsis microalgae", JOURNAL OF THE TAIWAN INSTITUTE OF CHEMICAL ENGINEERS, vol. 67, 2016, pages 106-114, XP002783302,
- TAHER ET AL: "Effective extraction of microalgae lipids from wet biomass for biodiesel production", BIOMASS AND BIOENERGY, vol. 66, 2014, pages 159-167, XP002783303,
- FANG ET AL: "Extraction and demulsification of oil from wheat germ, barley germ, and rice bran using an aqueous enzymatic method", JOURNAL OF THE AMERICAN OIL CHEMISTS' SOCIETY, vol. 91, 2014, pages 1261-1268, XP002783318,

## Description

The present invention refers to a method for extracting a triglyceride, a sugar and/or an amino acid from an oleaginous microorganism. Further, it is directed to the use of an enzyme system comprising an enzyme having hydrolase activity in such method.

### Technical background

In the past decade, the global demand for plant- and animal-based lipids as platform for pharmaceutical, food, biofuel and oleochemical industry increased by 65%. To meet the increase of market demand with taking into consideration the environmental aspects as to mitigate the CO₂ emissions motivate industry to develop novel sustainable bioprocesses.

With an ability to accumulate up to 70% lipids of their dry weight, oleaginous microorganisms are poised to be the next generation for host lipid production. The conversion of microorgansim's lipids into bio-kerosene, bio-diesel and bio-lubricants as well pharmaceutical and food applications has been well documented.

For example *Trichosporon oleaginosus* (*T. oleaginosus*; ATCC 20509), first isolated in 2011, shows a high ability to uptake varied monomeric carbon sources of pentoses and hexoses as well as complex waste material feedstocks. Under stress conditions, such as nitrogen, phosphate or sulfate limitation, *T. oleaginosus* converts these C-sources into triacylglycerol lipids, which are subsequently stored in sub-cellular droplets. In addition, commonly used oleaginous microorganisms exhibit fast growth rates and the fatty acid profile is close to that of the vegetable oils.

Biofuel, as yet, is occupied the main focusing of the single cell oil (SCO) production. Conversely, creating biopharmaceutical-based applications, so called red biotechnology of SCO has not well established so far. A very recent report, has expressed that SCO can be a valuable source of food-additive PUFAs. Görner *et al.* presented the engineering of *T. oleaginosus* for enhanced production of long-chain unsaturated fatty acids, such as eicosatrienoic, eicosadienoic and α-linolenic acid (Görner, C., et al., Genetic engineering and production of modified fatty acids by the non-conventional oleaginous yeast Trichosporon oleaginosus ATCC 20509. Green Chemistry, 2016, 18(7): p. 2037-2046). Moreover, Devyani *et al.* confirm that *Lipomyces starkey* can produce a PUFAs through submerged fermentation (Salunke, D., et al., Production of polyunsaturated fatty acids in recombinant Lipomyces starkeyi through submerged fermentation. Bioprocess and biosystems engineering, 2015, 38(7): p. 1407-1414).

Under stress conditions, oleaginous microorganisms generate lipid (triglyceride) droplets (LD) as storage compartments for triacylglycerols. The ultrastructure of lipid droplets consists of a phospholipid monolayer that contains bound proteins, which enables attachments to other cell compartments that represents a stable emulsifier system. Furthermore, a rigid cell-wall renders the cells resistant to many solvents, which complicates downstream lipid extraction procedures.

Conventional triglyceride extraction procedures require two discrete consecutive steps: The initial step comprises cell-wall lysis either via temperature shocks, chemical treatment or high pressure homogenization. In the second step triglycerides are extracted with organic solvent such as petroleum ether, methanol, or chloroform. None of these methods are easily scaled to industrial level due to cost issues and lack of environmental sustainability. Moreover, the latter application of organic solvents negatively impacts the quality of the finished product. Hence, the complexity of solvent removal is considered as extra cost and energy squandering.

Enzymatic approaches have previously been applied to enhance the cell rupture of oleaginous yeasts. To that end enzyme-assisted lipid release and subsequent ethyl acetate lipid extraction has recently been reported for *Rhodosporidium toruloides.* In analogy, a process termed enzyme-assisted aqueous extraction processing (EAEP) was applied to lipid extraction in the microalgae *Chlorella vulgaris, Scenedesmus dimorphus,* and *Nannochloropsis sp.* In the EAEP, a pretreatment of ultrasonic irradiation and posttreatment of heating at 95 °C was required. These pre-/post-treatments considered as a high-energy consuming process thus it is inapplicable at industrial scale. In 2011, a mix of different enzymes papain, pectinase, snailase, neutrase, alcalase and cellulase were optimized on the filamentous fungus *Mortierella alpina* for arachidonic acid extraction. To support the hydrolysis procedure, an addition of hexane was still required. Overall published processes, various pretreatments have been applied prior to the enzymatic cell lysis. Moreover, the lipids still have to be extracted with an organic solvent to recover the triglycerides.

It is of great interest to gain triglycerides of high purity and high rate of yield from microorganisms also due to problematic agricultural gaining of oil such as palm oil. It is divisive because palm oil was initially touted as a "green" crop and literally a fuel in making products less harmful to the atmosphere. The oil is a key component in biodiesel, considered a potential replacement for fossil fuel-based gasoline as an energy source for automobiles.

WO 2010/138620 discloses methods for the extraction of triglycerides from microbial biomass using an organic solvent and/or surfactants. WO 2002/10423 describes the extraction of microbial oil by cell lysis using enzymatic, physical or mechanical methods and separation of the oil, preferably polyunsaturated fatty acids (PUFA), via centrifugation. WO 2003/092628 refers to methods for the extraction of PUFAs from a plant or microorganism using surfactants Zheng et al. (Algal Research 2016, vol. 14, p. 100-108) discloses the use of amine co-polymers for increasing enzymatic cell wall disruption.

Hence, there is a need for an efficient, environmentally compatible, low-energy consuming method for gaining a triglyceride which method is suitable for small scale as well as industrial applications.

For the first time the present invention provides a single step pretreatment recovery process for single microorganism oils based on enzymatic lysis of an oleaginous microorganism followed by a simple demulsification. The process is scalable from lab to technical scale without causing any major investments.

### Summary

The present invention is as defined in the claims. It refers to a method for extracting a triglyceride from an oleaginous microorganism comprising the steps:
a) adding an enzyme system to the oleaginous microorganism, wherein the enzyme system comprises hydrolase activity to hydrolyze the cell wall of the microorganism,
b) optionally adding a protease, which is for example a serine protease or a group thereof, to the hydrolyzed oleaginous microorganism, and
c) adding a short-chain polymer, which is for example linear, branched or cross-linked, to the hydrolyzed oleaginous microorganism of step a) and/or b) for demulsification, wherein three separate layers are formed, one comprising the triglyceride, a second comprising cell debris and a third comprising sugar and/or amino acids, and
d) optionally isolating the triglyceride layer, which comprises for example short-chain, medium-chain or long-chain triglycerides or a combination thereof

The oleaginous microorganism is for example a fungi, yeast, bacteria or algae such as microalgae for example selected from the group consisting of *Trichosporon oleaginosus, Trichosporon capitatu, Trichosporon asahii, Lipomyces starkeyi, Rhodosporidium toruloides, Yarrowia lipolytica, Rhodotorula graminis, Rhodotorula glutinis, Cutaneotrichosporon oleaginosus, Apiotrichum curvarum, Cryptococcus curvatus, Candida sp, Rhodotorula gracilis, Chlorella sp., Pseudochlorococcum sp., Nannochloris sp, Nannochloropsis sp., Isochrysis sp, Tribonema minus, Dunaliella sp., Ankistrodesmus sp., Botryococcus braunii, Pavlova sp., Scenedesmus sp., Skeletonema sp., Nitzschia sp., Rhodococcus opacus, Acinetobacter calcoaceticus* and *Bacillus alcalophilus.*

The hydrolase activity of the enzyme system used in the method of the present invention is selected from the group consisting of cellulase, hemicellulase, mannase, laminarinase, glucanase, galacturonase, polygalacturonase, pectinase, glucosidase and a combination thereof.

The pH of step a) is for example in the range of 3 to 7, in the range of 4 to 6 or in the range of 4 to 5, and the temperature T is for example in the range of 30 °C to 70°C, in the range of 40 °C to 55 °C or in the range of 40 °C to 45 °C for any of the pH ranges.

The pH of step b) is for example in the range of 6 to 10, in the range of 7 to 9 or 8, and the temperature T is for example in the range of 25 °C to 45°C, in the range of 30 °C to 40 °C or in the range of 35 °C to 37 °C for any of the pH ranges.

The short-chain polymer used according to the present invention is selected from the group consisting of polyglycol, polyadduct of polyglycol and alkenyl succinic acid anhydride, polyester of polyglycol, alkenyl succinic anhydride polyester, block copolymer of propylenoxide and ethylenoxide, polyglycerol, polyester of poly-α-olefine and a combination thereof.

The triglycerides isolated by the method of the present invention comprise for example palmitic acid (C_{16:0}), stearic acid (C_{18:0}), oleic acid (C_{18:1}), linoleic acid (C_{18:2}), α-linolenic acid (C_{18:3}), arachidic acid (C_{20:0}), arachidonic acid (C_{20:4}), eicosapentaenoic acid (C_{20:5}), behenic acid (C_{22:0}), lignoceric acid (C_{24:0}) or a combination thereof.

In addition to or alternatively to isolating the triglyceride layer, the sugar layer is isolated, wherein the sugar is for example selected from the group consisting of glucuronic acid, rhamnose, fucose, mannitol, xylose, mannose, glucose and a combination thereof, optionally comprising an amino acid which is further isolated.

The present invention further refers to the use of an enzyme system comprising an enzyme having hydrolase activity as defined in claim 1, in a method of the present invention.

All documents cited or referenced herein ("herein cited documents"), and all documents cited or referenced in herein cited documents, together with any manufacturer's instructions, descriptions, product specifications, and product sheets for any products mentioned herein or in any document incorporated by reference herein, may be employed in the practice of the invention.

### Description of figures

The following figures show the invention in more detail, however, the invention is not limited to these figures.

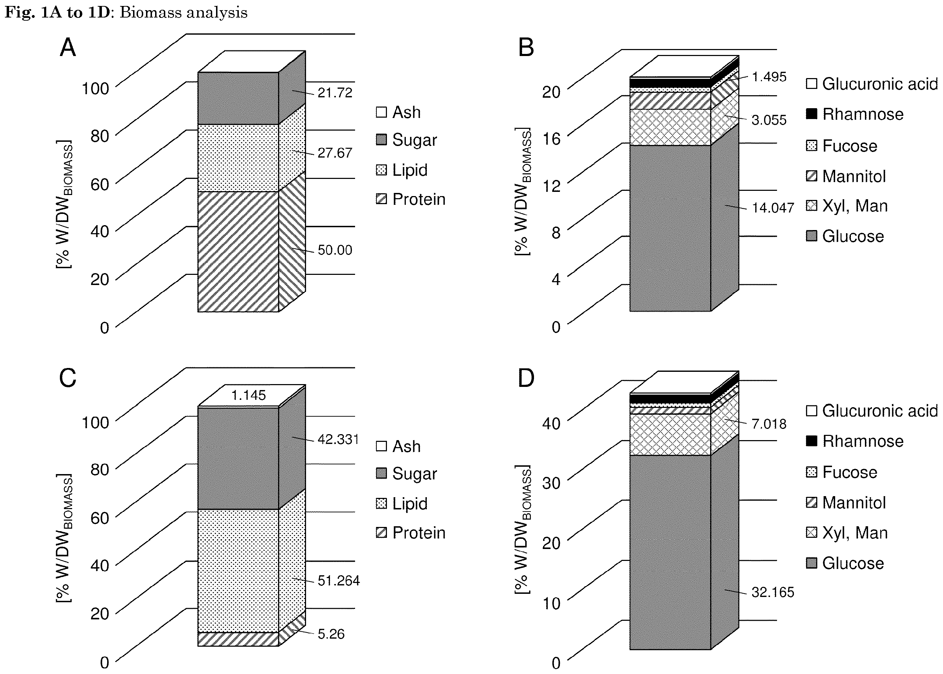

**Figures 1A and 1C** depict the biomass composition of *Trichosporon oleaginosus*, cultivated for 5 days at 28 °C. **Figures 1B** and **1D** show sugar compositions of *Trichosporon oleaginosus via* chemical hydrolysis at 2.0, 3.0, 10.0 [% v/v] H₂SO₄, for 60 min at 121 °C. The media used are YPD media in **Figures 1A** and **1B** and minimal Nitrogen Media in **Figures 1C** and **1D**.

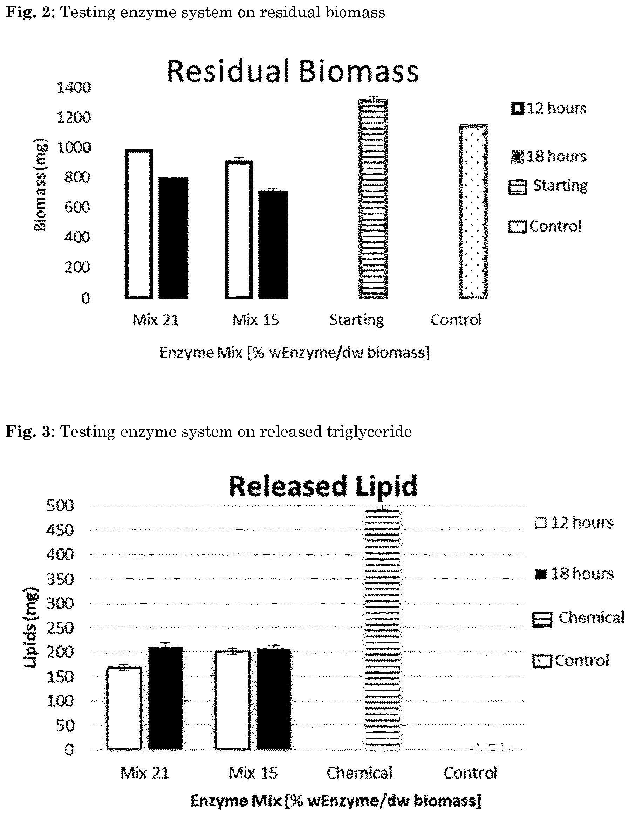

**Figure 2** shows a residual biomass of treated *T. oleaginosus* with mix 15 (comprising mannanase, Cellic Ctec2, Cellic Htec and beta-glucosidase) and mix 21 (comprising Liquebeet, CLA, mannanase, 1,3-beta-glucanase and beta-glucosidase). Cells were incubated for 12 hours (white) and 18 hours (black) at pH 5.0 (Acetate buffer, 50.0 mM), 55.0 °C with 250 rpm. Starting (striped): the amount of biomass at the starting of reaction, Control 2 (dotted) biomass was incubated at same condition without any enzyme additions.

**Figure 3** depicts a released triglyceride of treated *T. oleaginosus* with mix 15 (comprising mannanase, Cellic Ctec2, Cellic Htec and beta-glucosidase) and mix 21 (comprising Liquebeet, CLA, mannanase, 1,3-beta-glucanase and beta-glucosidase). Cells were incubated for 12 hours (white) and 18 hours (black) at pH 5.0 (Acetate buffer, 50.0 mM), 55.0 °C with 250 rpm. Used controls: Chemical (striped) the extracted triglyceride after high-pressure homogenizer followed by solvent extraction. Control (dotted) biomass was incubated at same condition without any enzyme additions.

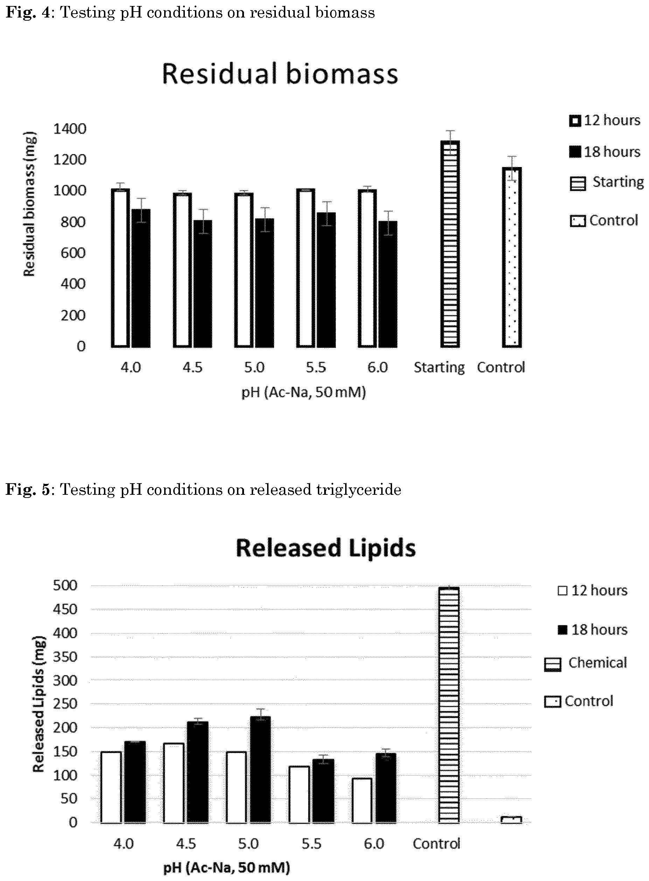

**Figure 4** depicts a residual biomass of treated *T. oleaginosus* at different pH conditions which were pH 4.0, 4.5, 5.0, 5.5 and 6.0 (Acetate buffer, 50.0 mM). Cells were incubated with mix 21 (comprising Liquebeet, CLA, mannanase, 1,3-beta-glucanase and beta-glucosidase) for 12 (white) and 18 (black) hours at 55 °C and 250 rpm. Used controls: Starting (striped): the amount of biomass at the starting of reaction, Control 2 (dotted) biomass was incubated at same condition without any enzyme additions.
**Figure 5** shows released lipid of treated *T. oleaginosus* at different pH conditions which were 4.0, 4.5, 5.0, 5.5 and 6.0 (Acetate buffer, 50.0 mM). Cells were incubated with mix 21 (comprising Liquebeet, CLA, mannanase, 1,3-beta-glucanase and beta-glucosidase) for 12 (white) and 18 (black) hours at 55 °C and 250 rpm. Used controls: Chemical (striped) the extracted triglyceride after high-pressure homogenizer followed by solvent extraction, Control (dotted) biomass was incubated at same condition without any enzymes additions.

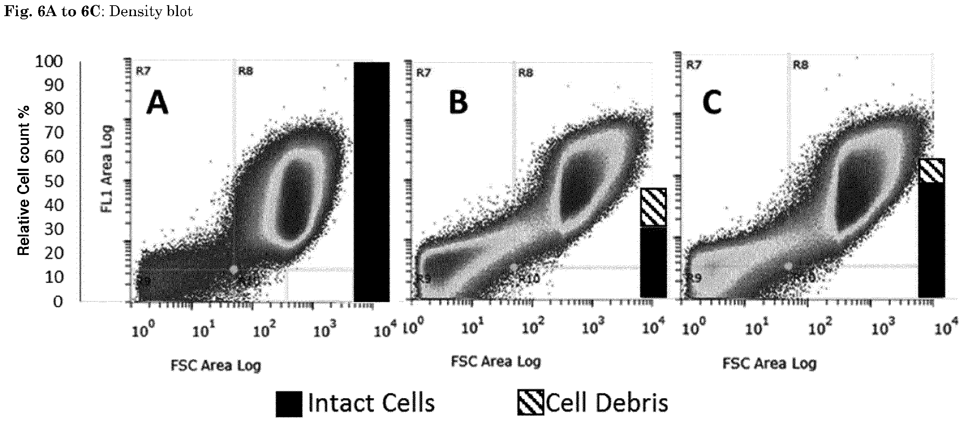

**Figures 6A - 6C** show the density blot and cell count of treated T. *oleaginosus* (logarithmic scaling of x- and y-axis is identical). **Figure 6A** refers to untreated samples (Control), **Figure 6B** to samples treated at pH 4.5 and **Figure 6C** to samples treated at pH 5.0 (Acetate buffer, 50.0 mM). Samples were incubated with mix 21 (comprising Liquebeet, CLA, mannanase, 1,3-beta-glucanase and beta-glucosidase) for 18 hours at 55 °C and 250 rpm.

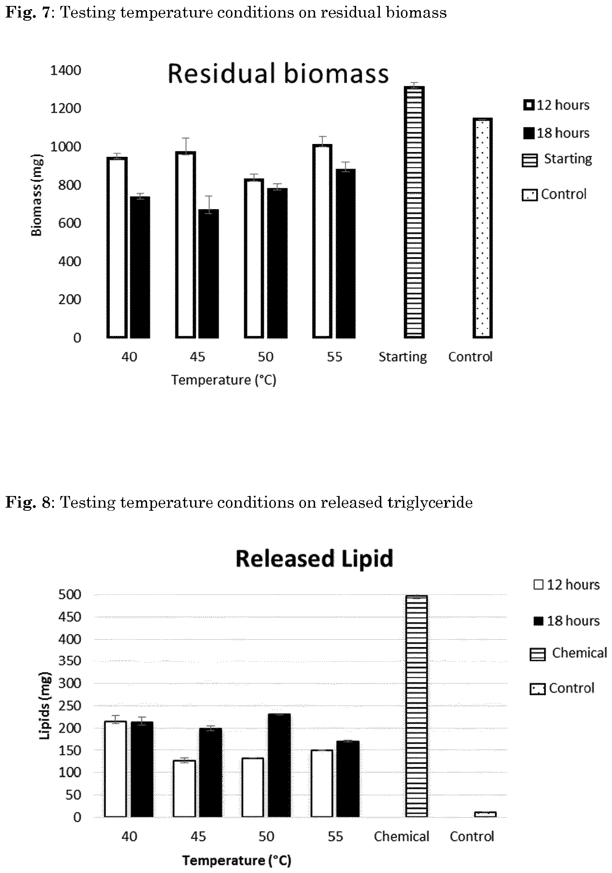

**Figure 7** depicts residual biomass of treated *T. oleaginosus* under different temperatures: 40, 45, 50 and 55°C. Cells were incubated with mix 21 (comprising Liquebeet, CLA, mannanase, 1,3-beta-glucanase and beta-glucosidase) for 12 (white) and 18 (black) hours, at pH 4.0 (Acetate buffer, 50.0 mM) with 250 rpm. Used controls: Starting (striped): the amount of biomass at the starting of the reaction, Control 2 (dotted) biomass was incubated at same condition without any enzyme additions.
**Figure 8** depicts released triglyceride of treated *T. oleaginosus* under different temperatures: 40, 45, 50 and 55°C. Cells were incubated with mix 21 (comprising Liquebeet, CLA, mannanase, 1,3-beta-glucanase and beta-glucosidase) for 12 (white) and 18 (black) hours, at pH 4.0 (Acetate buffer, 50.0 mM) with 250 rpm. Used controls: Chemical (striped) the extracted triglyceride after high-pressure homogenizer followed by solvent extraction, Control (dotted) biomass was incubated at same condition without any enzyme additions.

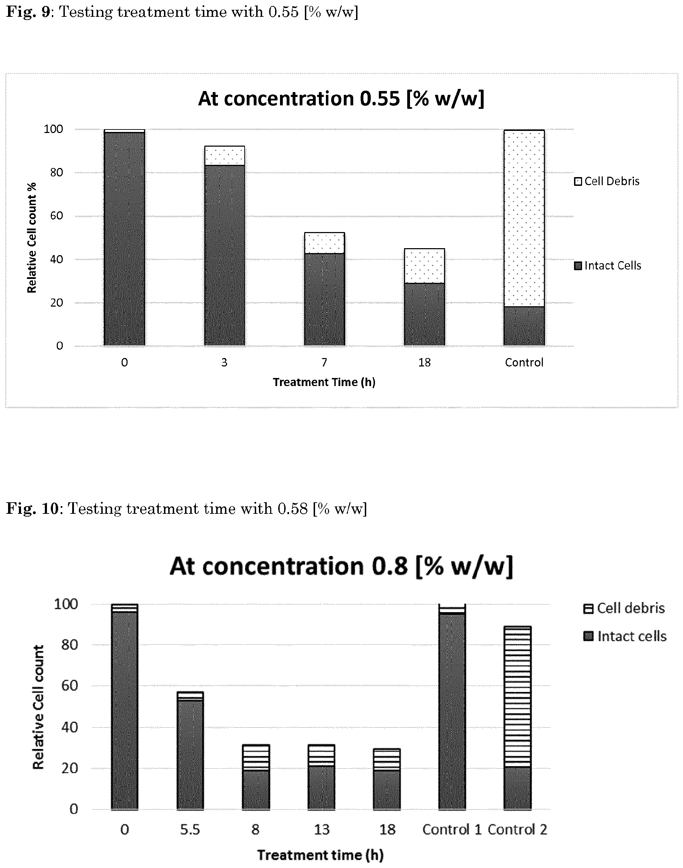

**Figure 9** shows the relative cell count of treated T. *oleaginosus* at different incubation periods (0, 3, 7 and 18 h) with Mix 21 (comprising Liquebeet, CLA, mannanase, 1,3-beta-glucanase and beta-glucosidase) at 0.55 [% w/w]. Cells were incubated at pH 4.5 and a temperature of 45.0 °C, the control refers to treatment with 3 times high-pressure homogenizer.
**Figure 10** depicts the relative cell count of treated *T. oleaginosus* at different incubation periods (0, 5.5, 8, 13 and 18 h) with Mix 21 (comprising Liquebeet, CLA, mannanase, 1,3-beta-glucanase and beta-glucosidase) at 0.8 [% w/w]. Cells were incubated at pH 4.5 and a temperature of 5.0 °C. Control 1 is an untreated sample, Control 2 refers to treatment with 3 times high-pressure homogenizer.

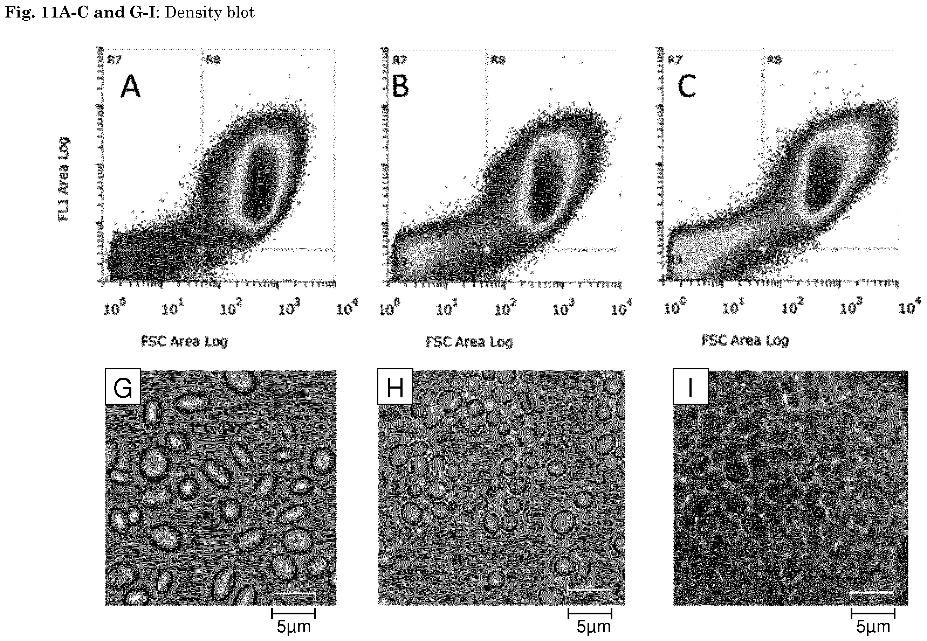

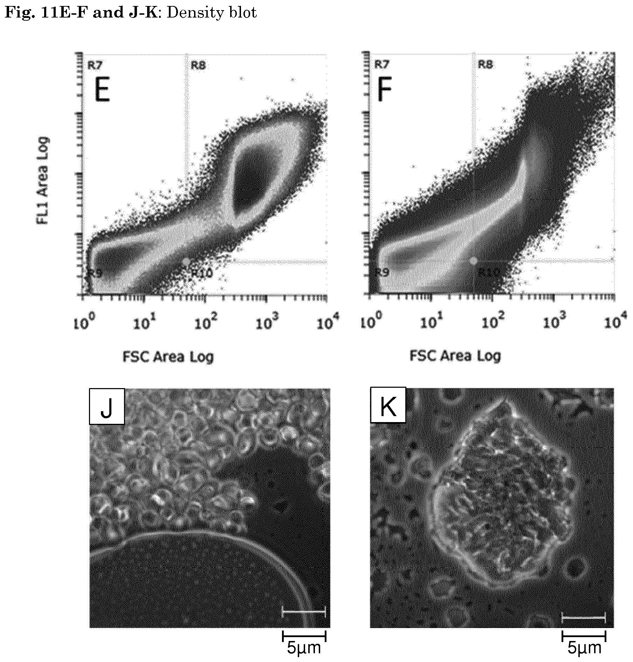

**Figures 11A - 11F** show the density blot of treated *T. oleaginosus* at different incubation time periods (0, 3, 7 and 18 h) with Mix 21 (comprising Liquebeet, CLA, mannanase, 1,3-beta-glucanase and beta-glucosidase) at 0.55 [% w/w]. Cells were incubated at pH 4.5 and a temperature of 45.0 °C. The control refers to treatment with 3 times high-pressure homogenizer. **Figures 11G - 11K** depict the corresponding fluorescence microscope observation at a microscope magnification of 100x and exposure time of 39 m sec. Logarithmic scaling of x- and y-axis in **Fig. 11A-11C****,** **11E** and **11F** is identical.

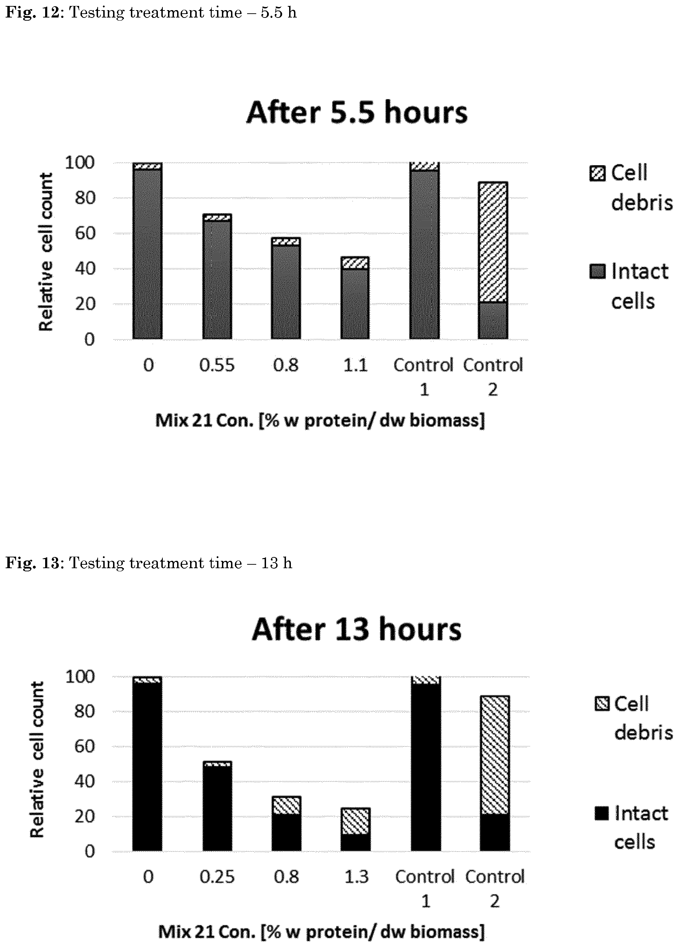

**Figure 12** shows the relative cell count of treated *T. oleaginosus* at different concentrations (0, 0.55, 0.8 and 1.1 [% wₚᵣₒₜₑᵢₙ / dw_{biomass}]) of Mix 21 (comprising Liquebeet, CLA, mannanase, 1,3-beta-glucanase and beta-glucosidase) for 5.5 h. Cells were incubated at pH 4.5 and a temperature of 45.0 °C. Control 1 refers to an untreated sample, Control 2 to the treatment with 3 times high-pressure homogenizer.
**Figure 13** depicts the relative cell count of treated *T. oleaginosus* at different concentrations (0, 0.25, 0.8 and 1.3 [% wₚᵣₒₜₑᵢₙ / dw_{biomass}]) of Mix 21 (comprising Liquebeet, CLA, mannanase, 1,3-beta-glucanase and beta-glucosidase) for 13 h. Cells were incubated at pH 4.5 and a temperature of 45.0 °C. Control 1 refers to an untreated sample, Control 2 to the treatment with 3 times high-pressure homogenizer.

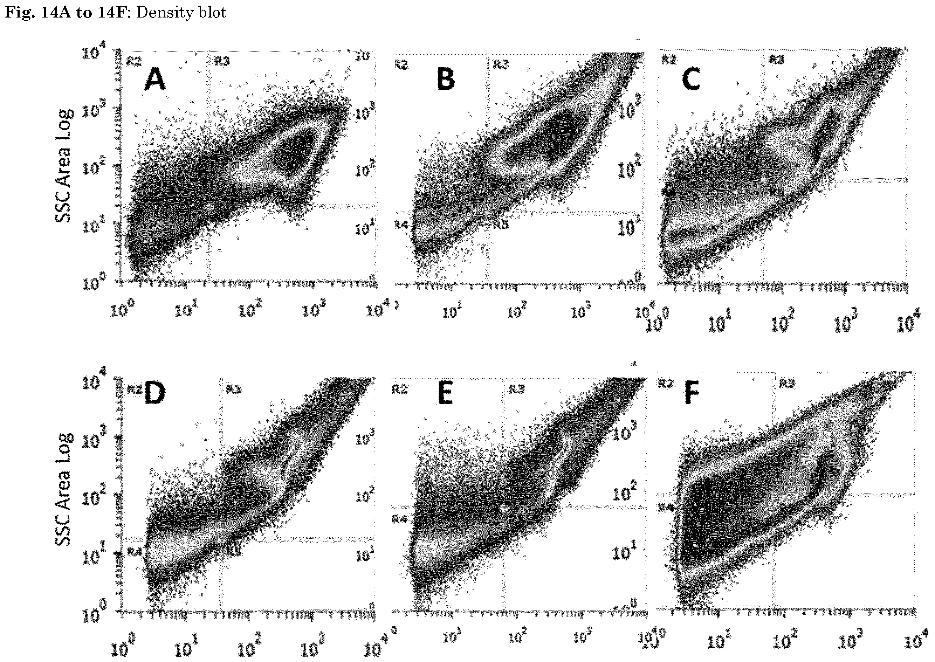

**Figures 14A - 14F** depict the density blot of treated *T. oleaginosus* at different concentrations of Mix 21 (comprising Liquebeet, i.e., a glycoside hydrolase, CLA, mannanase, 1,3-beta-glucanase and beta-glucosidase): **Fig. 14A****:** 0.0 [% w/w], **Fig. 14B****:** 0.55 [% w/w], **Fig. 14C****:** 0.8 [% w/w], **Fig. 14D****:** 1.1 [% w/w], **Fig. 14E****:** 1.3 [% w/w], and **Fig. 14F****:** 3x Avestin (Control). Cells were incubated for 13 hours at pH 4.5, 45 °C and 250 rpm.

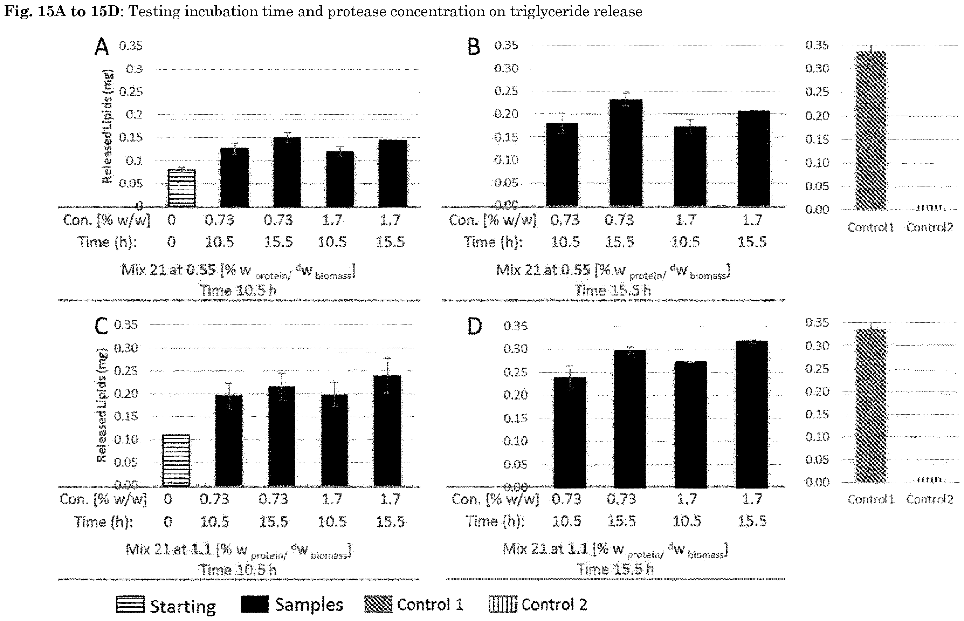

**Figures 15A - 15D** show released triglyceride of *T. oleaginosus* treated with glycoside hydrolase and Mix 21 (comprising Liquebeet, i.e., a glycoside hydrolase, CLA, mannanase, 1,3-beta-glucanase and beta-glucosidase), respectively, at different times (0, 10.5 and 15.5 h) and protease concentrations (0, 0.73 and 1.7 [% wₚᵣₒₜₑᵢₙ / dw_{biomass}]), transverse striped column is without protease addition. Cells were incubated at pH 4.5 and a temperature of 45.0 °C for cell-lysis. Protease treatment was conducted at pH 8.0 and a temperature of 37.0. Control 1 (checked) refers to the extracted lipid after high-pressure homogenizer followed by solvent extraction, Control 2 (longitudinally striped) to the untreated biomass.

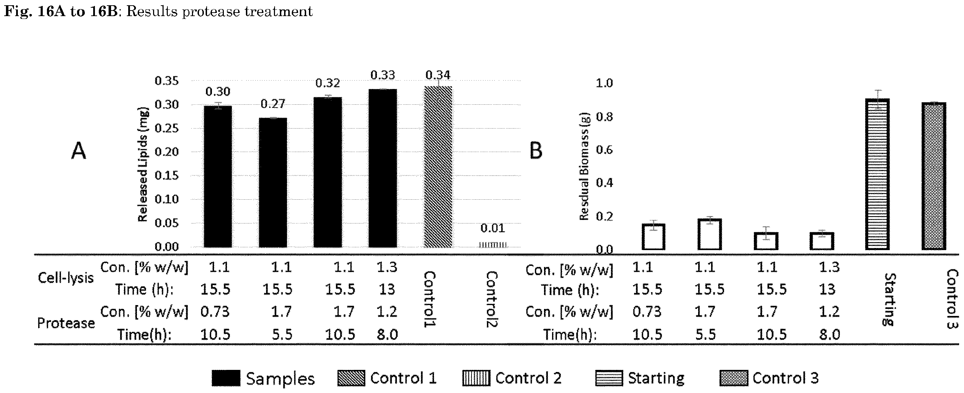

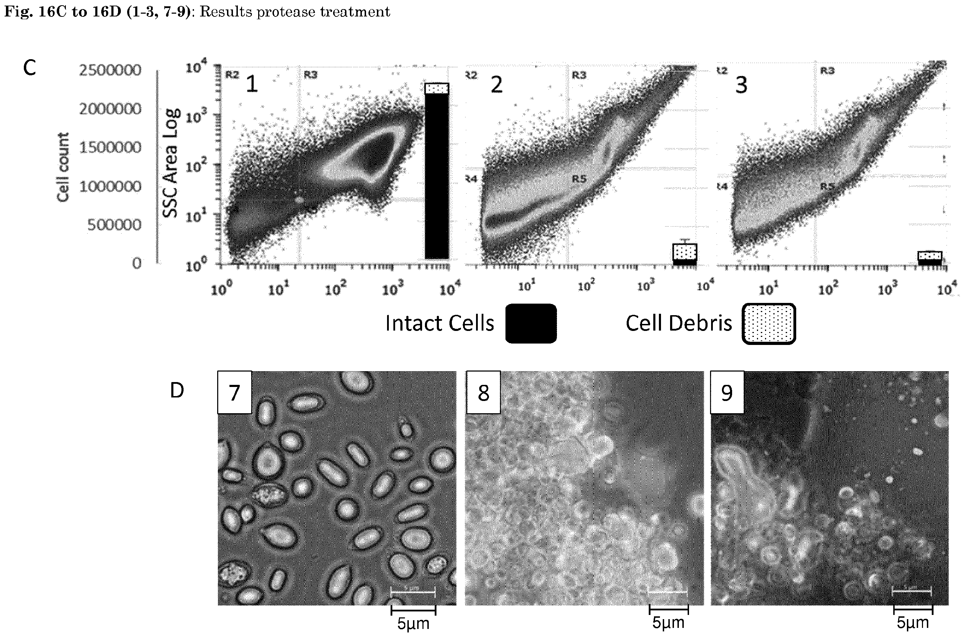

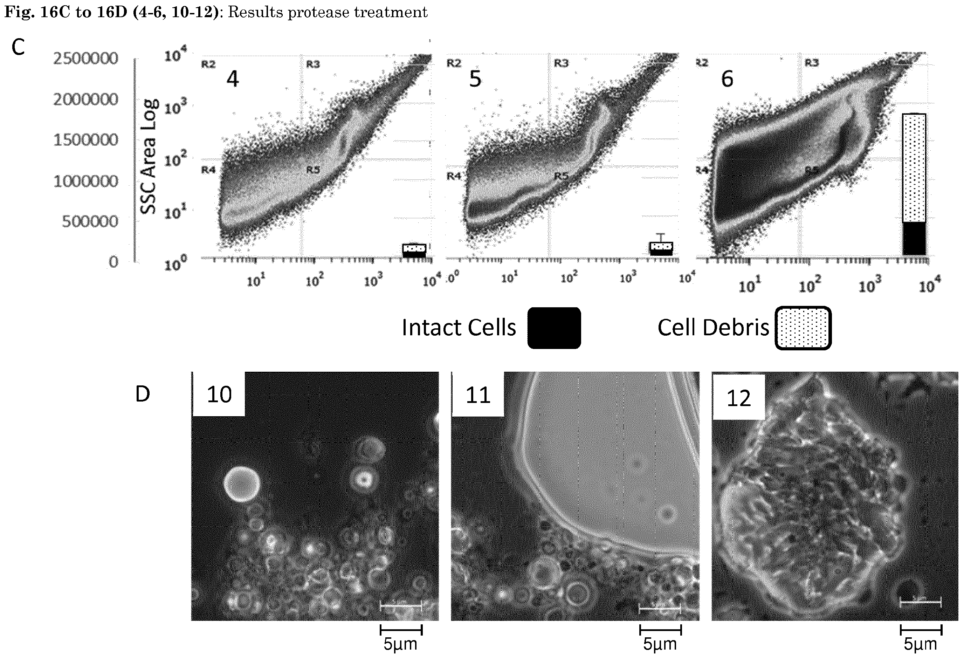

**Figures 16A** - **16D** show released lipid (**Fig. 16A**), residual biomass (**Fig. 16B**), density blot and cell counts (**Fig. 16C**) and corresponding fluorescence microscope observation of treated *T. oleaginosus* at different time points (13 and 15.5 h for cell-lysis and 5.5, 8.0 and 10.5 h for protease treatment) and enzymes concentrations (1.1 and 1.3 [% w/w] for cell-lysis and 0.73, 1.2 and 1.7 [% w/w] for protease treatment; **Fig. 16D**). Cells were incubated at pH 4.5 and a temperature of 45.0 °C for cell-lysis. Protease treatment was conducted at pH 8.0 and a temperature of 37.0. Control 1 (in green) refers to the extracted triglyceride after high-pressure homogenizer followed by solvent extraction, Control 2 (in orange) refers to untreated biomass. Microscope magnification of 100x at exposure time 39 m sec.

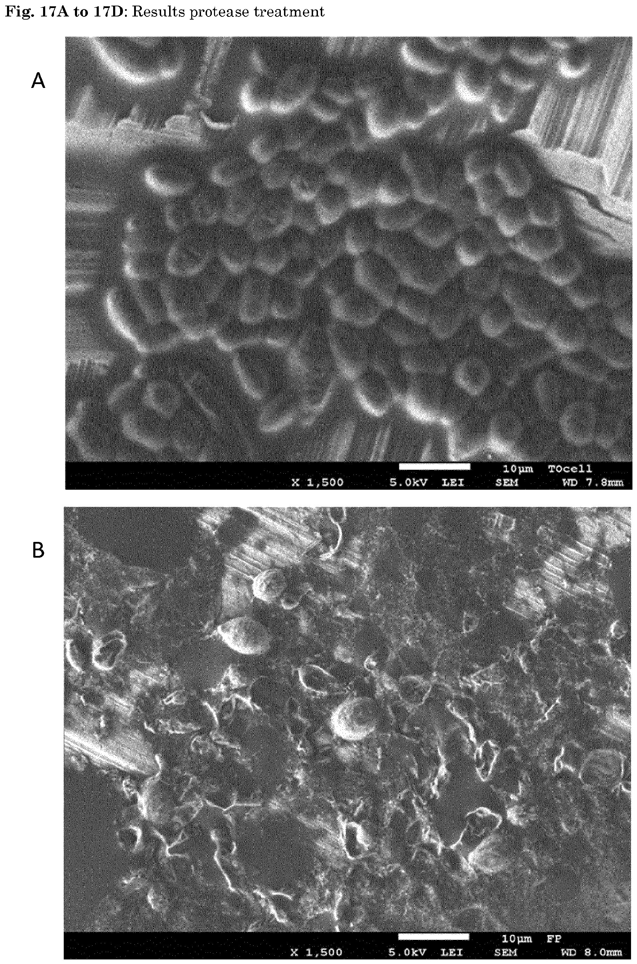

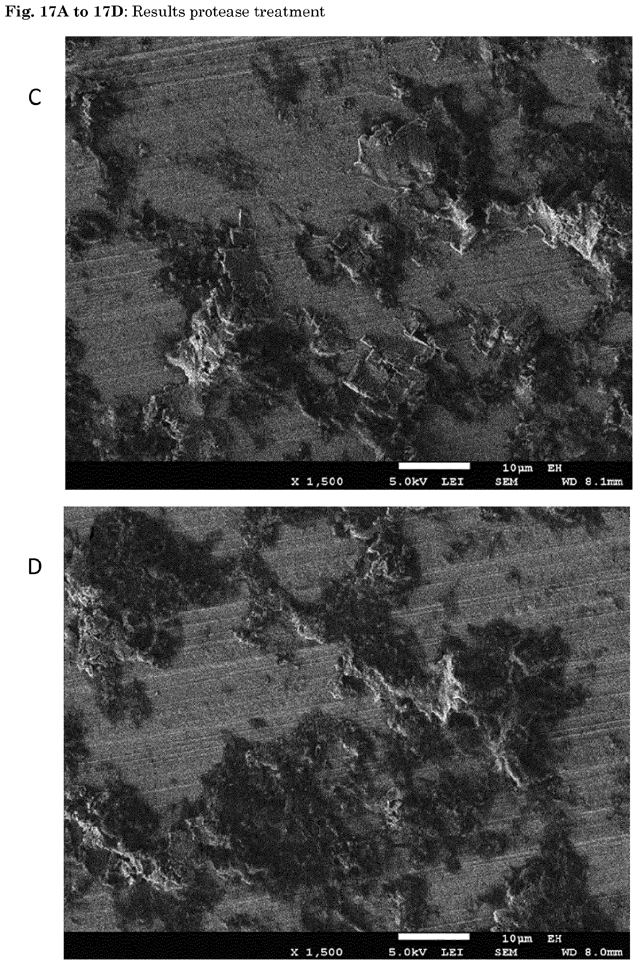

**Figures 17A - 17D** show electron microscope scatter for untreated cell (**Fig. 17A**), TO after treatment with 3x Avestin (**Fig. 17B**), *T. oleaginosus* after cell lysis step (**Fig. 17C**) and after protease treatment (**Fig. 17D**). Magnification of 1500 x, 5.0 KV with SEM detector.

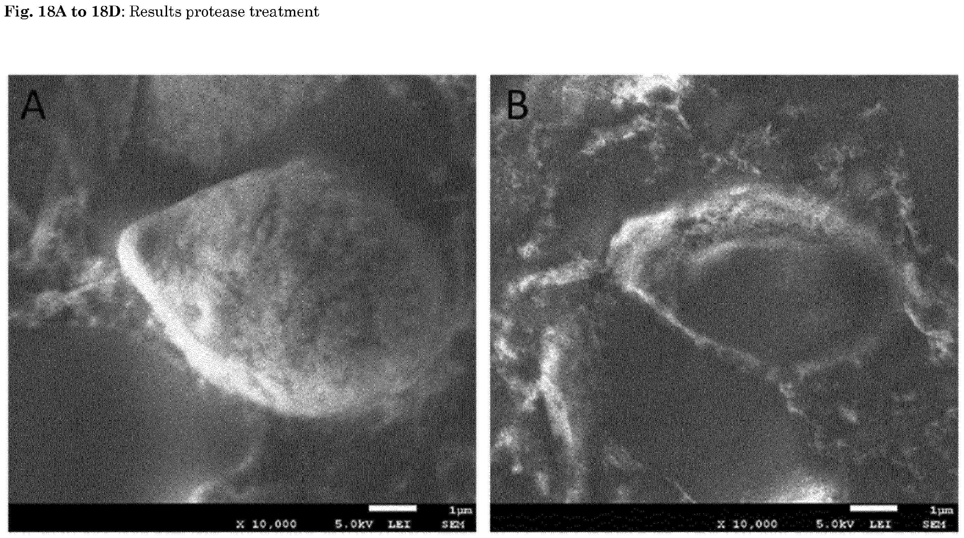

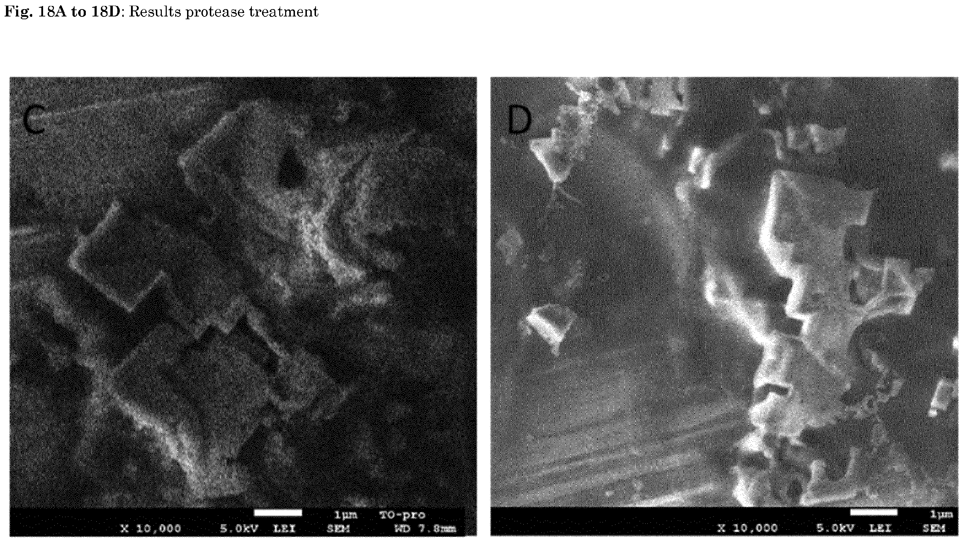

**Figures 18A - 18D** depict electron microscope photos for untreated cells (**Fig. 18A**), *T*. *oleaginosus* after treatment with 3x Avestin (**Fig. 18B**), *T. oleaginosus* after cell lysis step (**Fig. 18C**) and after protease treatment (**Fig. 18D**). Magnification of 10000 x, 5.0 KV with SEM detector.

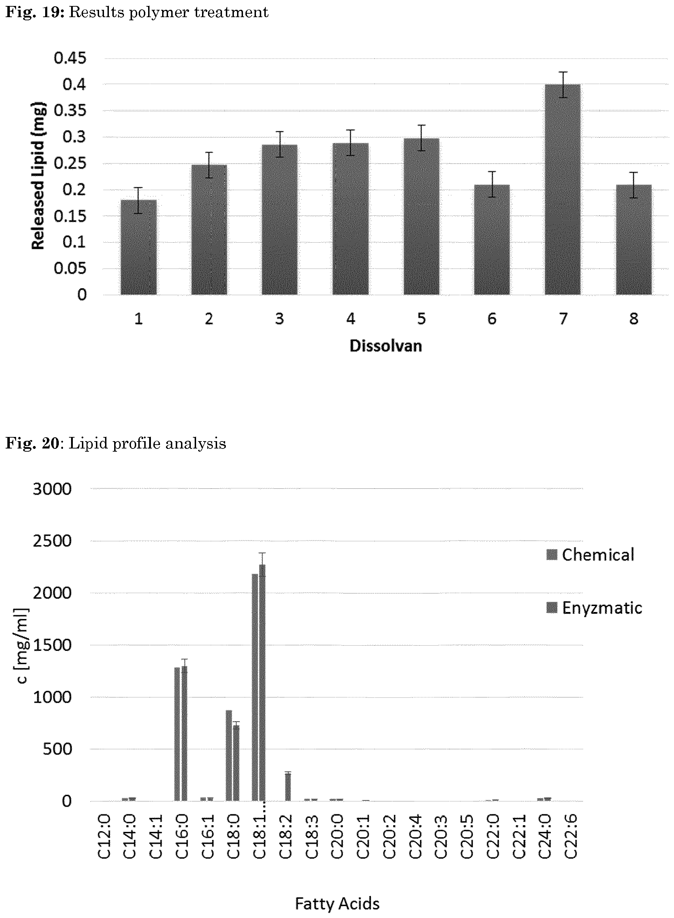

**Figure 19** shows released triglyceride after addition of different short-chain polymers (dissolvan). Cells were incubated at pH 4.5 and a temperature of 45.0 °C with Mix 21 for cell-lysis. Protease treatment was conducted at pH 8.0 and a temperature of 37.0 °C. Used short-chain polymers: **1.** Polymer of mixed alkoxylat-ethyl-glycerol, **2.** Propoxylated EO/PO block polymer-bis glycidyl ether with excess of EO, **3.** Propoxylated EO/PO block polymer-bis glycidyl ether (low, < 50 % PO), **4.** Polypropylenglycol of high MW with excess of EO (> 30 %), **5.** Cross-linked PEG, **6.** Poly-α olefin-polyester (active ingredient > 80%), **7.** Poly-α olefin-polyester (active ingredient > 98%) and 8. Propoxylated EO/PO block polymer-bis glycidyl ether (high, > 50 % PO).
**Figure 20** depicts the fatty acid profiles of extracted triglycerides via (blue) chemical method and (red) enzymatic method. The fatty acid profile was quantified using GC-FID after methylation under acid and base conditions. C19 TAG was used as internal standard for methylation efficiency.

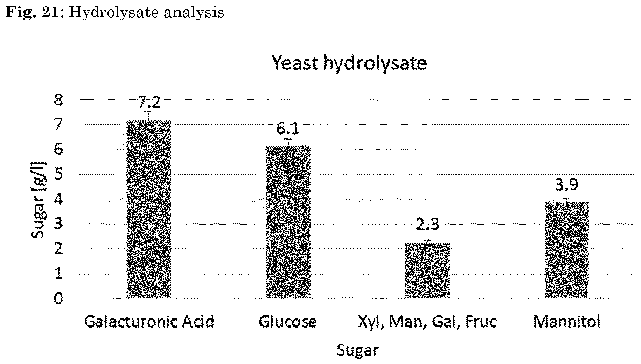

**Figure 21** shows the sugar profile of hydrolysis of the cell-wall polysaccharides of *T. oleaginosus.*

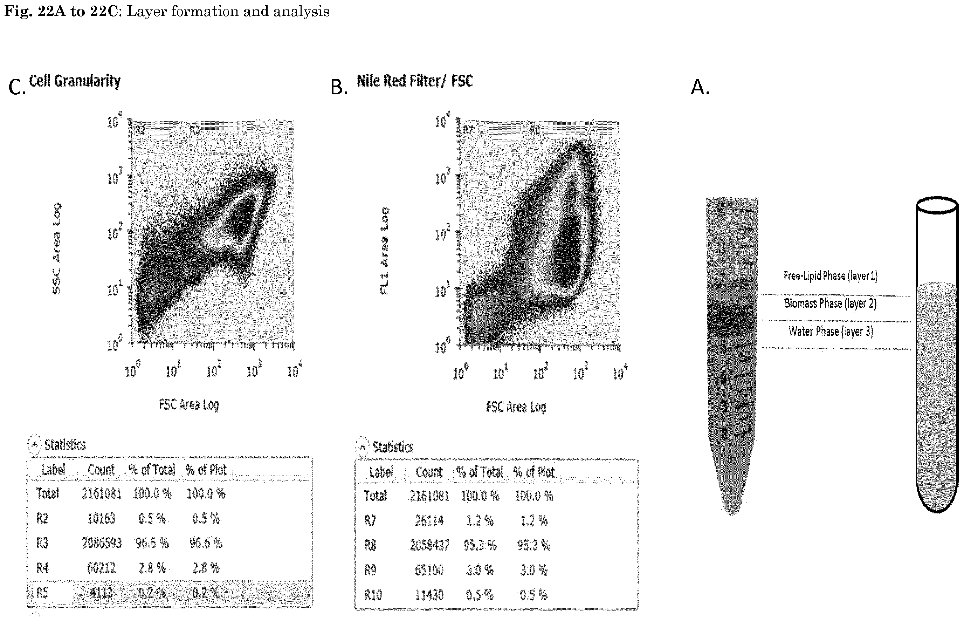

**Figures 22A - 22C** depict the releasing of triglyceride layer on the top of the reaction tube (**Fig. 22A**). The separation was obtained via centrifugation at 9000 g for 20 min. The density blot, "Y" axis is fluorescence at 588±24 nm and "X" axis is forward scatter (**Fig. 22B**) and the density blot, "Y" axis is side scatter and "X" axis is forward scatter (**Fig. 22C**).

### Detailed description

The present invention is directed to a novel downstream process for triglyceride recovery from oleaginous microorganisms such as bacteria, fungi, yeast and algae, e.g. microalgae, with low energy consumption and a recovery rate of up to 99.9 % for example 90, 91, 92, 93, 94, 95 96, 97, 98, 99 or 99.9 % of the total accumulated lipid in the microorganism with a method completely free of the use of a solvent. Cell lysis is based on enzymatic treatment using an enzyme system comprising for example a combination of enzymes having a synergistic lysis effect. In addition, the method of the present invention comprises the addition of a short-chain polymer as defined in claim 1, to the hydrolyzed oleaginous microorganism resulting in the formation of a triglyceride layer and a cell debris layer and a sugar layer optionally comprising an amino acid.

In the following, the elements of the present invention will be described in more detail. These elements are listed with specific embodiments, however, it should be understood that they may be combined in any manner and in any number to create additional embodiments falling within the scope of the claims. The variously described examples and embodiments should not be construed to limit the present invention to only the explicitly described embodiments. This description should be understood to support and encompass embodiments which combine the explicitly described embodiments with any number of the disclosed elements. Furthermore, any permutations and combinations of all described elements in this application should be considered disclosed by the description of the present application unless the context indicates otherwise and as long as they fall within the scope as defined in the claims.

Throughout this specification and the claims, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps. The terms "a" and "an" and "the" and similar reference used in the context of describing the invention (especially in the context of the claims) are to be construed to cover both the singular and the plural, unless otherwise indicated herein or clearly contradicted by the context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as", "for example"), provided herein is intended merely to better illustrate the invention and does not pose a limitation on the scope of the invention otherwise claimed. No language in the specification should be construed as indicating any non-claimed element essential to the practice of the invention.

The present invention refers to a solvent free, e.g., *in-situ* method for direct release of lipids from oleaginous microorganisms. The method is characterized by moderate conditions regarding temperature with low energy consumption based on enzymatic hydrolysis of the cell-wall followed by a demulsification step comprising breaking the emulsifier system of the cell via protease treatment and treatment with a short-chain polymer of the residual biomass to recover pure lipids after mild centrifugation or separation, e.g., using a milk-separator or milk-separator like device. In a side aspect, the method of the present invention creates operating costs in total about ten times less than operating costs of classical methods.

One of the key challenges for the scalability of single cell lipid production is the need to implement an industrial-applicable downstream process for lipid recovery with low energy consumption and an additional criteria for food and pharmaceutical application is the recovery of a triglyceride and optionally other end-products such as sugar and/or amino acids free from any traces of organic solvent.

The method of the present invention relates to extracting a triglyceride from an oleaginous microorganism which is a bacteria, fungi, yeast or algae such as a microalgae. A bacteria is for example *Rhodococcus opacus, Acinetobacter calcoaceticus* or *Bacillus alcalophilus, a fungi is for example Mucor circinelloides* and *Mortierella alpine, Aspergillus oryzae* or *Humicola lanuginose, a yeast is for example Lipomyces starkeyi, Rhodosporidium toruloides, Yarrowia lipolytica, Rhodotorula graminis, Rhodotorula glutinis, Cutaneotrichosporon oleaginosu,. Apiotrichum curvarum, Cryptococcus curvatus, Candida sp, Rhodotorula gracilis, Trichosporon capitatum* or *Trichosporon asahii* and an algae such as a microalgae is for example *Chlorella sp., Pseudochlorococcum sp., Nannochloris sp, Nannochloropsis sp., Isochrysis sp, Tribonema minus, Dunaliella sp., Ankistrodesmus sp., Botryococcus braunii, Pavlova sp. , Scenedesmus sp., Skeletonema sp.,* or *Nitzschia sp..* The oleaginous microorganism used in the present invention is for example only one single type of oleaginous microorganism, a mixture of different oleaginous fungi, yeast, bacteria or algae such as microalgae, or a mixture of different oleaginous fungi, yeast, bacteria and algae such as microalgae.

The method of the present invention, as defined in claim 1, comprises or consists of the steps:
a) adding an enzyme system to the oleaginous microorganism, wherein the enzyme system comprises an enzyme having hydrolase activity to hydrolyze the cell wall of the microorganism,
b) adding a protease to the hydrolyzed oleaginous microorganism, and
c) adding a short-chain polymer to the hydrolyzed oleaginous microorganism of step b) for demulsification, wherein three separate layers are formed, one comprising the triglyceride, a second comprising cell debris and a third comprising sugar and/or amino acids. The steps a) to c) are performed separately one after the other, or all compounds added in steps a) to c), steps a) and b), steps b) and c) or steps a) and c) are added to the oleaginous microorganism at once. In case of separate steps a) to c) the time between the steps takes seconds, minutes, hours or days, and the time between the steps is identical or differs.

In step c) one or more short-chain polymer(s) are added, i.e., the short-chain polymer is one type of short-chain polymer or a mixture of different short-chain polymers in different ratios.

In step c) different layers are formed: one comprising or consisting of triglyceride, one comprising or consisting of sugar and/or amino acid and one comprising or consisting of cell debris. The layers are separate and comprise or consist of the triglyceride, sugar and/or amino acid or cell debris from about 50 % to 100 %, 60 % to 99 %, 70 % to 98 %, 80 % to 97 %, 90 % to 96 %, or 95 %, 96 %, 97 %, 98 %, 99 %, 99.9 % or 100 % (purity). The layers are formed for example by centrifugation, e.g., using a milk-separator or a milk-separator-like device. In an optional step d) the triglyceride layer, the sugar layer and/or amino acid layer and/or the layer of cell debris are isolated, e.g., by detracting via a syringe. The isolated triglyceride, sugar, amino acid and/or cell debris may be further purified or treated or directly used in food or fuel production.

The enzyme system used in the method of the present invention comprises or consists of any enzyme having one or more hydrolase activity/ies, wherein the enzymes of the enzyme system have a synergistic hydrolase activity effect. Enzymes of the enzyme system are a cellulase, a hemicellulase, a mannase, a laminarinase, a glucanase, a galacturonase, a polygalacturonase, a pectinase, a glucosidase or combinations thereof. Concentrations of the enzyme system used in the method of the present invention are for example 0.1 to 5, 0.2 to 4.5, 0.3 to 4.0, 0.4 to 3.5, 0.5 to 3.0, 0.6 to 2.5 or 0.5 to 2.7 [% wₚᵣₒₜₑᵢₙ/dw_{biomass}], which is percent weight of the protein bzw. enzyme / dry weight of the biomass.

The design of a specific enzyme system is essential for cell lysis and oil recovery. The enzyme system is for example optimized on a biomass such as an artificial biomass in order to carry out a complete cell lysis into monosaccharaides. In the first stage of optimization for example, the biochemical components of a partially purified cell-wall of a well-established oleaginous microorganism such as *T. oleaginosus* is analyzed. Data resulting from biomass analysis are for example used for the selection of required enzyme activates for selective cell lysis of the well-established oleaginous microorganism. Subsequently, the optimized enzyme system is tested for example directly on harvested culture of living cells without any pretreatment. Process conditions of pH, incubation temperature, enzyme concentration and space-time conversion yields are for example evaluated in order to minimize the process cost and duration. For the demulsification of the cell lysate, a protease activity is for example added to the enzyme system to accomplish a direct release of single cellular lipid. FACS cell counting in combination with fluoresce-/ electron- microscope and gravimetric analysis are used for example to evaluate the efficiency of the newly established process. Finally, for example triglyceride extraction at scale of 2 liters is performed to verify the scalability of the suggested downstream process.

In the method of the present invention the pH is controlled in the different method steps. In step a) the pH is for example in the range of 3 to 7, in the range of 4 to 6 or in the range of 4 to 5, or the pH is 2, 3, 4, 5, 6, 7 or 8. In step b) the pH is for example in the range of 6 to 10, in the range of 7 to 9 or the pH is 5, 6, 7, 8 or 9. The pH is increased or decreased with any type of acidic or basic compound.

Optionally, the temperature is controlled in the different method steps independent of the pH. In step a) the temperature T is for example in the range of 30 °C to 70°C, in the range of 40 °C to 55 °C or in the range of 40 °C to 45 °C, or 30, 35, 40, 45, 50, 55, 60, 65 or 70 °C. In step b) the temperature T is for example in the range of 25 °C to 45°C, in the range of 30 °C to 40 °C or in the range of 35 °C to 37 °C, or 25, 30, 31, 32, 33, 34, 35, 36 37, 38, 39, 40 or 45 °C.

The method of the present invention comprises a demulsification, wherein a protease is added to the hydrolyzed oleaginous microorganism. The protease is one type of protease, a group of the same type of protease or a combination of different types of proteases. The different types of proteases are serine protease, cysteine protease, threonine protease, aspartic protease, glutamic protease, metalloprotease and asparagine peptide lyase, respectively.

The demulsification further comprises the addition of a short-chain polymer, i.e., a low molecular weight polymer (e.g., MW about 10.000 g/mol to about 20.000 g/mol such as < 20.000 g/mol, <15.000 or < 10.000 g/mol), in particular a polycondensate. The polymer used in a method of the present invention breaks for example a water-in-oil-emulsion or an oil-in-water-emulsion. The polymer is for example linear, branched or cross-linked. Said polymers are polyglycol, polyadduct of polyglycol and alkenyl succinic acid anhydride, polyester of polyglycol, alkenyl succinic anhydride polyester, block copolymer of propylenoxide and ethylenoxide, polyglycerol, polyester of poly-α-olefine or shown in the following **Table 1** or a combination thereof:

| **Polymer** | **Active ingredient** |
|---|---|
| Propoxylated EO*/PO** block polymer - bis glycidyl ether (high, > 50 % PO), e.g., 1 conc. | e.g., > 97 % |
| Propoxylated EO/PO- block polymer bis-glycidyl ether (low, < 50 % PO), e.g., 1 conc. | e.g., 100 |
| Polypropylenglycol of high MW with excess of EO (> 30 %), e.g., 1 conc. | e.g., 100 |
| Propoxylated EO/PO block polymer - bis glycidyl ether (high, > 50 % PO) with an excess of EO, e.g., 1 conc. | e.g., 100 |
| Cross-linked PEG, e.g., 1 conc. | e.g., > 79 % |
| Polymer of mixed alkoxylat-ethyl-glycerol, e.g., 1 conc. | e.g., 100 |
| Poly- α olefin- polyester | e.g., 80 or 98 % |

| | |
|---|---|
| *EO - ethylenoxide **PO - propylenoxide | |

The polymer may be dissolved in a solvent such as S. Naphtha or 2- Ethylhexanol.

After the demulsification the oleaginous microorganism treated according to the method of the present invention is allowed to stand for separation of the different layers comprising or consisting of a triglyceride, a sugar and/or an amino acid, and/or cell debris, or it is subjected to a, e.g., mechanical, separation step using a centrifuge or a separator such as a milk-separator or milk-separator-like device.

The triglyceride extracted and isolated, respectively, by a method of the present invention comprises or consists of for example a short-chain, a medium-chain or a long-chain triglyceride or a combination thereof. The triglyceride comprises any saturated and/or unsaturated fatty acid for example palmitic acid (C_{16:0}), stearic acid (C_{18:0}), oleic acid (C_{18:1}), linoleic acid (C_{18:2}), α-linolenic acid (C_{18:3}), arachidic acid (C_{20:0}), arachidonic acid (C_{20:4}), eicosapentaenoic acid (C_{20:5}), behenic acid (C_{22:0}) and/or lignoceric acid (C_{24:0}). The sugar extracted and isolated, respectively, by a method of the present invention comprises or consists of any sugar for example selected from the group consisting of glucuronic acid, rhamnose, fucose, mannitol, xylose, mannose, glucose and a combination thereof.

The amino acid extracted and isolated, respectively, by a method of the present invention comprises or consists of any amino acid depending on the protease(s) used for the demulsification. The amino acid is in a separate layer in a purity of 90, 91, 92, 93, 94, 95, 96, 97, 98 or 99 % or in mixture with the sugar for example in an aqueous phase. In the latter case, the amino acid may be separated from the sugar in one or more additional purification steps.

The method of the present invention optionally comprises an additional step f) for recycling the compounds used in the method of the present invention for example the enzyme system, the protease and/or the polymer.

The present invention further refers to the use of an enzyme system comprising an enzyme having hydrolase activity as defined in claim 1, in a method of the present invention.

The description further discloses a kit comprising an enzyme system and optionally a carrier or support system and/or a device such as a reaction tube for example to perform a method of the present invention for extraction of a triglyceride.

### Examples

The following examples illustrate different embodiments of the present invention, but the invention is not limited to these examples.

### Example 1: Biomass analysis

Glucuronoxylomannan (GXM) has been reported to be the expressed in the cell walls of some *Trichosporon spp.,* i.e. *Trichosporon beigelii, Trichosporon asahii* and *Trichosporon cutaneum.* GXM is a 1,3-linked mannan backbone attached to short side chains of 1,4-linked mannose and 1,2-linked xylose residues. Depending on the strain type, the ratio of this polysaccharide to the protein is varied in the cell membrane. **Fig. 1A** shows the overall biomass analysis of the *T. oleaginosus.* The cell wall displays a high amount of protein content about 50 % and relatively low lipid content of 15 %, while the total carbohydrates was nearly 27 %. However, no ash was detectable after incineration at 1000 °C.

The compositional sugars consist mainly of glucose, mannose, xylose, rhamnose and mannitol (**Fig. 1B**). The cell-wall composition is altered when the *T. oleaginosus* is cultivated in Minimal-N media. **Fig. 1C** shows a decrease in the protein content to be 5.3 %. Contrariwise, sugar, ash and lipid were increased up to 42.3, 1.5 and 51.3 % respectively. However, the sugar profile was almost identical.

### Example 2: Investigation of enzyme system

An enzyme system was tested that is able to hydrolyze the cell-wall of the *T. oleaginosus* without the need to any pretreatment. Additionally, the more monomeric sugars are produced via the enzyme system, the better the process since the sugars are considered as an advantageous side product which can be utilized in further applications. Data obtained of the biomass analysis (**Fig. 1A** - **1D**) define the necessary enzyme activities like cellulases, hemicellulases, mannanases, laminarases and a *β*-glucosidase.

In the optimization phase, two criteria were used for the differentiation between 48 various enzyme mixtures at varied concentrations. These criteria are the increase of sugar concentration and decrease of residual biomass weight. Two exemplary mixtures (Mix 15 and Mix 21) showed good activity within an acceptable time frame and adequate concentration. Mix 15 contains Novozymes enzyme products Mannanase, Cellic Ctec2, Cellic Htec and β-glucosidase, while Mix 21 contains Clariant enzymes Liquebeet, CLA, Mannanase, 1.3-β-glucanase and β-glucosidase. Both enzyme mixes showed almost the same activity with less than 30 [% w/w] residual biomass and about 15 g/l glucose after 3 days. The used enzyme mixture concentration was 1.4 [% w_{enzyme}/dw_{biomass}].

The *T. oleaginosus* was cultivated in minimal nitrogen media for 5 days. Afterwards, the biomass was harvested then divided into 15-ml falcon tubes. Each falcon tube was treated under different conditions of pH, temperature, concentration, and time. After incubation, the analysis was done as described in the materials and methods.

### Example 3: Optimization of Enzyme Mix

As first step, the best two mixtures were tested on harvested culture. The conditions mentioned in proof of concept were used in this experiment. However, the analysis was done at two time intervals which were 12 and 18 h.

**Fig. 2** and **Fig. 3** show the residual biomass and released lipid, respectively. 50 [% w/w] of the lipid was moved out to the upper layer in the falcon tube. At the same ratio of lipid release, the biomass was decreased also about [50 % w/w]. Both mixes showed almost the same activity. Hence, in the further studies the Mixture 21 was used.

### a) pH optimization

Sometimes the increase of released triglyceride showed a symmetric result with the decrease of the biomass. Alternatingly, the pH of the reaction from 4.0 to 6.0 (at 55 °C) showed no significant effect on the decrease of the biomass (**Fig. 4**). Conversely, pH 4.5 and 5.0 showed the best triglyceride release results. **Fig. 6** shows the intact cell after treatment at pH 4.5 and 5.0 corresponding to 29.2 and 48.1 % of stating cell count respectively. Therefore, pH 4.5 is considered advantageous for this process.

### b) Temperature optimization

Increasing temperature from 40.0 to 55.0 °C (pH 4.0, 50.0 mM) showed a remarkable effect on the residual biomass rather than its impact on lipid release. Treatment at 40 °C resulted in more lipid within a shorter time period (**Fig. 8**). By increasing the time, 50 °C showed slight preponderance. In different way, lowest value of residual biomass was recorded at 45 °C (**Fig. 7**). Thus, temperatures of 40- 45 °C showed better results for the cell lysis step.

### c) Optimization of the enzyme concentration

The duration of the cell lysis and the enzyme load have a serious effect on the cost and the process efficiency. Generally, the more enzyme is used, the less time is required and *vice versa.* Thus, a range of 0.25 - 1.4 [% wₚᵣₒₜₑᵢₙ/dw_{biomass}] within a time period of 3 -18 h (at pH 5.0 and 55.0 °C) was selected. The best blend was evaluated by cell count associated with fluorescence microscopy.

**Fig. 9** and **Fig. 10** show the decrease of total cell count by increasing the treatment time at two enzyme concentrations: Low enzyme concentration of 0.55 [% w/w] intact-cell count dwindle down to about 40 % and 30 % after 7 and 8 hours, respectively. If the concentration is increased to 0.8 [% w/w], about 80 % of total cell count were degraded within 8 hours.

**Fig. 11** shows the cell density destruction gotten by FACS vs. obtained photos of the same treatment time. Staining with Nile Red confirmed the increase of cell aggregation by increasing treatment time. However, a big red spot of released lipid (stained with Nile Red) appeared after 18 h compared to homogenized mass when the samples is treated with Avestin for 3 times (**Fig. 11K**). Moreover, it can be seen from the microscope scatter, that those which appear at intact-cell region of the cell density diagrams are actually aggregated together (Figure **11H**, **11I** and **11J**) which is an indication that cells are effect by the cell lysis treatment.

Furthermore, **Fig. 12** and **Fig. 13** show the impact of the enzyme concentration on the degree of the cell lysis. After short treatment time (**Fig. 12**, 5.5 h) 50% of the cells are destructed if a 1.1 [% w_{enzyme}/dw_{biomass}] is loaded. Nevertheless, a rate of 80 and 90% destructive cell can be obtained if the treatment continue to 13 h at concentration of 0.8 and 1.3 [% w_{enzyme}/dw_{biomass}] respectively. Figure 14 shows the fading of the cell density in the area of intact cells.

### Example 4: Investigating Demulsification Step

Demulsification treatment consists of two phases: the first phase is addition of protease while the second refers to the addition of a short-chain polymer.

Previous work showed that the optimal pH and temperature for the selected protease is 8.0 and 37 °C, respectively (Data not shown). The optimization of these parameters cannot be separated from the cell-lysis step which has a significant impact on the demulsification step.

Hence, varied concentrations of short-chain polymers and different time intervals for incubation were studied. The samples were also treated at varied conditions of cell lysis to evaluate the impact of cell lysis step on the protease effectively.

**Fig. 15** shows the released lipid after different protease treatment for time intervals of 10.5 and 15.5 h at concentrations of 0.73 and 1.7 [% wₚᵣₒₜₑᵢₙ/dw_{biomass}]. Those samples were treated at different cell lysis conditions of time intervals (10.5 and 15.5 h) and concentrations (0.55 and 1.1 3 [% wₚᵣₒₜₑᵢₙ/dw_{biomass}]). The comparison of the diagrams shows a proportional increase of effectiveness of protease by increase of the intensity of cell wall lysis.

**Fig. 16** shows the best results for the cell lysis and protease treatment. The total time was in the range 20-25 h, while the total concentration was about 1.9-2.8 [%w/w]. The treatment was able to release up to 97 % to the total lipid comparing to the chemical method (**Fig. 16A**). These results are confirmed by the residual biomass measurements (**Fig. 16B**), insured FACS cell counting and fluorescence microscope observation (**Fig. 16C** and **16D**). **Fig. 17** and **18** show a comparison between Avestin and enzymatic cell lysis.

Additional experiments were performed with enzyme mix 21 to evaluate eight different types of short-chain polymers (1. Polymer of mixed alkoxylat-ethyl-glycerol, 2. Propoxylated EO/PO block polymer-bis-glycidyl ether with excess of EO, 3. Propoxylated EO/PO block polymer-bis-glycidyl ether (low, < 50 % PO) 4. Polypropylenglycol of high MW with excess of EO (> 30 %), 5. cross-linked PEG, 6. Poly-α olefin-polyester (active ingredient > 80 %), 7. Poly-α olefin-polyester (active ingredient > 98 %) and 8. Propoxylated EO/PO block polymer-bis-glycidyl ether (high, > 50 % PO). In this test, the biomass treated under different conditions of cell wall lysis and protease was collected, mixed and then divided equally into 8 tubes. Hence, 4 µl of each short-chain polymer was added into tubes, separately. After centrifugation, Poly-α olefin-polyester (active ingredient > 98 %) again provided the best result (**Fig. 19**).

The final product was subjected to GC FID to compare its lipid profile *vs.* the lipid profile extracted by the classical chemical procedure. **Fig. 20** displays the fatty acids consisting mainly of C_{16:0}, C_{18:0} C_{18:1} in addition to traces of other fatty acids. No significant variations were detected between the two profiles.

### Example 5: Hydrolysate analysis

HPLC (Agilent 1100 series) was used for the analysis of monomeric sugars. The HPLC was associated with Refractive Index [Shodex,RI101] and Ultraviolet Index [Sedere-France, Sedex75] as detectors. For best isolation of the moronic sugars, two different methods were used: firstly, a Rezex ROA-Organic Acid (Aminex HPX 87H) column was used with H₂SO₄ (5.0 mM) as mobile phase at a flow rate of 0.5 ml/min. The column was heated at 70°C, and the detector was set at 40 °C. Secondly, an Aminex HPX-87P column. An isocratic mobile phase of ddH₂O was pumped at a rate of 0.6 mL/min. Temperature was at 70 °C with the detector set at 50 °C.

The cell lysis via enzymatic-based approach has additional advantage. Hydrolysis of the polysaccharides of the cell wall lead to produce a fermentable sugars. **Fig. 21** shows the sugar analysis of the hydrolysate which contain mainly glucose, and galacturonic acid.

### Example 6: Cell lysis optimization

After harvesting the biomass, 1.0 or 0.5 g of lipid-accumulated biomass was distributed into 15 ml falcon tube. Later, 8-4 ml of acetate buffer (50.0mM) at different pH range 4.0 - 6.0. The reaction started by addition of enzyme solutions at different concentration 0.5-2.0 [% w_{enzyme}/w_{biomass}]. The reaction were incubated at different temperature 40.0-60.0 °C For varied incubation time 1-18 h.

To evaluate the effectively of the enzymes system on cell lysing and lipid releasing, three controls were used in parallel to the reactions: Control 1: A sample was treated with standard method of lipid extraction. The extracted amount of lipid was considered as 100% yield and it was used as reference to released lipids. Control 2: A sample contain the biomass and buffer. This sample incubated at the same conditions of temperature, pH, time and rotation without any addition of enzyme. Control 3: Point zero; sample was taken directly after addition of enzymes. Treatment time is 0 h.

Cell-lysis was followed by four assay methods. Samples were treated under different conditions were divided into two parts. The first part (1 ml) was used cell counting and microscope observation. The second part (the rest of samples) was centrifugation at 10,000 rpm for 20 min. after centrifugation three layers were formed (**Fig. 22A**). The lower layer, the water phase, was used for sugar analysis.

The upper layer was the released lipid. This layer was pipetted out into a known-weight 15 ml falcon tube for weight measurement.

The middle layer between the lipid and the water phases, was the sold residual biomass. This layer was subjected into known-weight 15 ml falcon tube for weight measurement after lyophilization under 0.0045 mbar at -80 °C for 2 days.

Cell Counting was performed using the FACS with laser beam of 488 nm and 100 mW. The counting was conducted using 100 µl of sample after 100 times dilution. **Fig. 22B** shows the cell density diagram "Y" axis is fluorescence at 588±24 nm and "X" axis is Forward scatter. **Fig. 22C** depicts the cell density diagram, "Y" axis is Side scatter and "X" axis is Forward scatter.

The Visual Observation was performed by florescence microscope after staining the samples with Nile red using 100x objector.

### Example 7: Lipid Extraction and Lipid Profile analysis

The standard method of lipid extraction is based on Bligh-Dyer's method (Bligh, E.G. and W.J. Dyer, A rapid method of total lipid extraction and purification. Canadian journal of biochemistry and physiology, 1959, 37(8): p. 911-917) as following: the biomass was treated with High-pressure homogenizer (Avestin emulsiflex C3) in order to disrupt the cell wall. Afterword, Folch Solution was used 3time to extract the lipids. Lipids amount were determinate gravimetrically.

The methylation of fatty acids (Dumay et al. 2006) (Griffiths et al. 2010) was performed in order to obtain the methyl ester derivatives for analysis by gas chromatography. 50.0 µl of lipid extract were diluted in 450 µl toluene. 50.0 µl (2mg/ml) Trinonadecnoin (C_{19:0}-TAG) were added prior to the reaction as an internal standard. Subsequently, 100 µl of 2,2-dimethoxypropane and 1.00 mL of sodium methoxide were added and the samples mixed briefly by vortexing before being placed in an incubator at 80 °C shaking at 900 rpm for 20 min. Samples were cooled for 5 min to room temperature and 1.00 mL HCl/methanol was added before repeating the incubation. After cooling for 5 min to room temperature, 400 µl be-distilled water and 360 µl n-hexane were added and the tubes mixed by vortexing. The samples were centrifuged at 4,000 rpm for 2 min and the upper hexane layer containing the FAME extract was transferred to vials for GC. Following lipid extracts were converted to FAMEs.

## Claims

1. Method for extracting a triglyceride from an oleaginous microorganism comprising the steps:
a) adding an enzyme system to the oleaginous microorganism, wherein the enzyme system comprises an enzyme having hydrolase activity to hydrolyze the cell wall of the microorganism and wherein the hydrolase activity is selected from the group consisting of cellulase, hemicellulase, mannase, laminarinase, glucanase, galacturonase, polygalacturonase, pectinase, glucosidase and a combination thereof,
b) optionally adding a protease to the hydrolyzed oleaginous microorganism, and
c) adding one or more short-chain polymer(s) to the hydrolyzed oleaginous microorganism of step a) and/or b) for demulsification, wherein three separate layers are formed, one comprising the triglyceride, a second comprising cell debris and a third comprising sugar and/or amino acids, and wherein the short-chain polymer is selected from the group consisting of polyglycol, polyadduct of polyglycol and alkenyl succinic acid anhydride, polyester of polyglycol, alkenyl succinic anhydride polyester, block copolymer of propylenoxide and ethylenoxide, polyglycerol, polyester of poly-α-olefine and a combination thereof
d) optionally isolating the triglyceride layer.

2. Method of claim 1, wherein the oleaginous microorganism is selected from the group consisting of *Trichosporon oleaginosus, Trichosporon capitatu, Trichosporon asahii, Lipomyces starkeyi, Rhodosporidium toruloides, Yarrowia lipolytica, Rhodotorula graminis, Rhodotorula glutinis, Cutaneotrichosporon oleaginous, Apiotrichum curvarum, Cryptococcus curvatus, Candida sp, Rhodotorula gracilis, Chlorella sp., Pseudochlorococcum sp., Nannochloris sp, Nannochloropsis sp., Isochrysis sp, Tribonema minus, Dunaliella sp., Ankistrodesmus sp., Botryococcus braunii, Pavlova sp., Scenedesmus sp., Skeletonema sp., Nitzschia sp., Rhodococcus opacus, Acinetobacter calcoaceticus* and *Bacillus alcalophilus.*

3. Method of any one of claims 1 or 2, wherein in step a) the pH is in the range of 3 to 7, in the range of 4 to 6 or in the range of 4 to 5.

4. Method of any one of claims 1 to 3, wherein in step a) the temperature T is in the range of 30 °C to 70°C, in the range of 40 °C to 55 °C or in the range of 40 °C to 45 °C.

5. Method of any one of claims 1 to 4, wherein in step b) the pH is in the range of 6 to 10, in the range of 7 to 9 or 8.

6. Method of any one of claims 1 to 5, wherein in step b) the temperature T is in the range of 25 °C to 45°C, in the range of 30 °C to 40 °C or in the range of 35 °C to 37 °C.

7. Method of any one of claims 1 to 6, wherein the short-chain polymer is linear, branched or cross-linked.

8. Method of any one of claims 1 to 7, wherein the triglyceride layer comprises short-chain, medium-chain or long-chain triglycerides or a combination thereof.

9. Method of any one of claims 1 to 8, wherein the triglycerides comprise palmitic acid (C_{16:0}), stearic acid (C_{18:0}), oleic acid (C_{18:1}), linoleic acid (C_{18:2}), α-linolenic acid (C_{18:3}), arachidic acid (C_{20:0}), arachidonic acid (C_{20:4}), eicosapentaenoic acid (C_{20:5}), behenic acid (C_{22:0}), lignoceric acid (C_{24:0}) or a combination thereof.

10. Method of any one of claims 1 to 9 comprising additional step e) isolating the sugar layer.

11. Method of any one of claims 1 to 10, wherein the sugar is selected from the group consisting of glucuronic acid, rhamnose, fucose, mannitol, xylose, mannose, glucose and a combination thereof.

12. Method of any one of claims 1 to 11, wherein the protease is a serine protease, a cysteine protease, a threonine protease, an aspartic protease, a glutamic protease, a metalloprotease and an asparagine peptide lyase or a combination thereof.

13. Use of an enzyme system comprising an enzyme having hydrolase activity as defined in claim 1, in a method according to any one of claims 1 to 12.

## Patentansprüche

1. Verfahren zum Extrahieren eines Triglycerids aus einem ölhaltigen Mikroorganismus, umfassend die Schritte:
a) Zugeben eines Enzymsystems zu dem ölhaltigen Mikroorganismus, wobei das Enzymsystem ein Enzym mit Hydrolaseaktivität umfasst, um die Zellwand des Mikroorganismus zu hydrolysieren, und wobei die Hydrolaseaktivität ausgewählt ist aus der Gruppe, bestehend aus Cellulase, Hemicellulase, Mannase, Laminarinase, Glucanase, Galacturonase, Polygalacturonase, Pectinase, Glucosidase und einer Kombination davon,
b) wahlweise Zugeben einer Protease zu dem hydrolysierten ölhaltigen Mikroorganismus, und
c) Zugeben von einem oder mehreren kurzkettigen Polymer(en) zu dem hydrolysierten ölhaltigen Mikroorganismus von Schritt a) und/oder b) zur Demulgierung, wobei drei getrennte Schichten gebildet werden, eine, umfassend das Triglycerid, eine zweite, umfassend Zelltrümmer, und eine dritte, umfassend Zucker und/oder Aminosäuren, und wobei das kurzkettige Polymer ausgewählt ist aus der Gruppe, bestehend aus Polyglycol, Polyaddukt von Polyglycol und Alkenyl-Bernsteinsäure-Anhydrid, Polyester von Polyglycol, Alkenyl-Bernsteinsäure-Anhydrid-Polyester, Block-Copolymer von Propylenoxid und Ethylenoxid, Polyglycerol, Polyester von Poly-α-Olefin und einer Kombination davon,
d) wahlweise Isolieren der Triglyceridschicht.

2. Verfahren nach Anspruch 1, wobei der ölhaltige Mikroorganismus ausgewählt ist aus der Gruppe, bestehend aus *Trichosporon oleaginosus, Trichosporon capitatu, Trichosporon asahii, Lipomyces starkeyi, Rhodosporidium toruloides, Yarrowia lipolytica, Rhodotorula graminis, Rhodotorula glutinis, Cutaneotrichosporon oleaginous, Apiotrichum curvarum, Cryptococcus curvatus, Candida sp, Rhodotorula gracilis, Chlorella sp., Pseudochlorococcum sp., Nannochloris sp, Nannochloropsis sp., Isochrysis sp, Tribonema minus, Dunaliella sp., Ankistrodesmus sp., Botryococcus braunii, Pavlova sp., Scenedesmus sp., Skeletonema sp., Nitzschia sp., Rhodococcus opacus, Acinetobacter calcoaceticus und Bacillus alcalophilus.*

3. Verfahren nach einem der Ansprüche 1 oder 2, wobei in Schritt a) der pH im Bereich von 3 bis 7, im Bereich von 4 bis 6 oder im Bereich von 4 bis 5 liegt.

4. Verfahren nach einem der Ansprüche 1 bis 3, wobei in Schritt a) die Temperatur T im Bereich von 30 °C bis 70 °C, im Bereich von 40 °C bis 55 °C oder im Bereich von 40 °C bis 45 °C liegt.

5. Verfahren nach einem der Ansprüche 1 bis 4, wobei in Schritt b) der pH im Bereich von 6 bis 10, im Bereich von 7 bis 9 oder 8 liegt.

6. Verfahren nach einem der Ansprüche 1 bis 5, wobei in Schritt b) die Temperatur T im Bereich von 25 °C bis 45 °C, im Bereich von 30 °C bis 40 °C oder im Bereich von 35 °C bis 37 °C liegt.

7. Verfahren nach einem der Ansprüche 1 bis 6, wobei das kurzkettige Polymer linear, verzweigt oder quervernetzt ist.

8. Verfahren nach einem der Ansprüche 1 bis 7, wobei die Triglyceridschicht kurzkettige, mittelkettige oder langkettige Triglyceride oder eine Kombination davon umfasst.

9. Verfahren nach einem der Ansprüche 1 bis 8, wobei die Triglyceride Palmitinsäure (C_{16:0}), Stearinsäure (C_{18:0}), Ölsäure (C_{18:1}), Linolsäure (C_{18:2}), α-Linolensäure (C_{18:3}), Arachinsäure (C2o:o), Arachidonsäure (C_{20:4}), Eicosapentaensäure (C_{20:5}), Behensäure (C_{22:0}), Lignocerinsäure (C_{24:0}) oder eine Kombination davon umfassen.

10. Verfahren nach einem der Ansprüche 1 bis 9, umfassend den zusätzlichen Schritt e) Isolieren der Zuckerschicht.

11. Verfahren nach einem der Ansprüche 1 bis 10, wobei der Zucker ausgewählt ist aus der Gruppe, bestehend aus Glucuronsäure, Rhamnose, Fucose, Mannitol, Xylose, Mannose, Glucose und einer Kombination davon.

12. Verfahren nach einem der Ansprüche 1 bis 11, wobei die Protease eine Serinprotease, eine Cysteinprotease, eine Threoninprotease, eine Asparaginsäureprotease, eine Glutaminsäureprotease, eine Metalloprotease und eine Asparagin-Peptid-Lyase oder eine Kombination davon ist.

13. Verwendung eines Enzymsystems, umfassend ein Enzym mit Hydrolaseaktivität, wie in Anspruch 1 definiert, in einem Verfahren nach einem der Ansprüche 1 bis 12.

## Revendications

1. Procédé pour extraire un triglycéride à partir d'un micro-organisme oléagineux comprenant les étapes consistant à :
a) ajouter un système d'enzyme au micro-organisme oléagineux, dans lequel le système d'enzyme comprend une enzyme possédant une activité hydrolase pour hydrolyser la paroi cellulaire du micro-organisme et dans lequel l'activité hydrolase est sélectionnée dans le groupe consistant en la cellulase, l'hémicellulase, la mannase, la laminarinase, la glucanase, la galacturonase, la polygalacturonase, la pectinase, la glucosidase et une combinaison de celles-ci,
b) facultativement ajouter une protéase au micro-organisme oléagineux hydrolysé, et
c) ajouter un ou plusieurs polymère(s) à chaîne courte au micro-organisme oléagineux hydrolysé de l'étape a) et/ou b) pour une démulsification, où trois couches distinctes sont formées, une comprenant le triglycéride, une deuxième comprenant des débris cellulaires et une troisième comprenant un sucre et/ou des acides aminés, et où le polymère à chaîne courte est sélectionné dans le groupe consistant en le polyglycol, un produit de polyaddition du polyglycol et d'un anhydride alkényle de l'acide succinique, un polyester de polyglycol, un polyester d'anhydride alkényle succinique, un copolymère séquencé d'oxyde de propylène et d'oxyde d'éthylène, le polyglycérol, un polyester de poly-α-oléfine et une combinaison de ceux-ci
d) facultativement isoler la couche du triglycéride.

2. Procédé selon la revendication 1, dans lequel le micro-organisme oléagineux est sélectionné dans le groupe consistant en *Trichosporon oleaginosus, Trichosporon capitatu, Trichosporon asahii, Lipomyces starkeyi, Rhodosporidium toruloides, Yarrowia lipolytica, Rhodotorula graminis, Rhodotorula glutinis, Cutaneotrichosporon oleaginous, Apiotrichum curvarum, Cryptococcus curvatus,* l'espèce *Candida, Rhodotorula gracilis,* l'espèce *Chlorella,* l'espèce *Pseudochlorococcum,* l'espèce *Nannochloris,* l'espèce *Nanochloropsis,* l'espèce *Isochrysis, Tribonema minus,* l'espèce *Dunaliella,* l'espèce *Ankistrodesmus, Botryococcus braunii,* l'espèce *Pavlova,* l'espèce *Scenedesmus,* l'espèce *Skeletonema,* l'espèce *Nitzschia, Rhodococcus opacus, Acinetobacter calcoaceticus* et *Bacillus alcalophilus.*

3. Procédé selon l'une quelconque des revendications 1 ou 2, dans lequel à l'étape a), le pH est dans la plage de 3 à 7, dans la plage de 4 à 6 ou dans la plage de 4 à 5.

4. Procédé selon l'une quelconque des revendications 1 à 3, dans lequel à l'étape a), la température T est dans la plage de 30 °C à 70 °C, dans la plage de 40 °C à 55 °C ou dans la plage de 40 °C à 45 °C.

5. Procédé selon l'une quelconque des revendications 1 à 4, dans lequel à l'étape b), le pH est dans la plage de 6 à 10, dans la plage de 7 à 9 ou 8.

6. Procédé selon l'une quelconque des revendications 1 à 5, dans lequel à l'étape b), la température T est dans la plage de 25 °C à 45 °C, dans la plage de 30 °C à 40 °C ou dans la plage de 35 °C à 37 °C.

7. Procédé selon l'une quelconque des revendications 1 à 6, dans lequel le polymère à chaîne courte est linéaire, ramifié ou réticulé.

8. Procédé selon l'une quelconque des revendications 1 à 7, dans lequel la couche du triglycéride comprend des triglycérides à chaîne courte, à chaîne moyenne ou à chaîne longue ou une combinaison de ceux-ci.

9. Procédé selon l'une quelconque des revendications 1 à 8, dans lequel les triglycérides comprennent l'acide palmitique (C_{16:0}), l'acide stéarique (C_{18:0}), l'acide oléique (C_{18:1}), l'acide linoléique (C_{18:2}), l'acide α-linolénique (C_{18:3}), l'acide arachidique (C_{20:0}), l'acide arachidonique (C_{20:4}), l'acide éicosapentaénoïque (C_{20:5}), l'acide béhénique (C_{22:0}), l'acide lignocérique (C_{24:0}) ou une combinaison de ceux-ci.

10. Procédé selon l'une quelconque des revendications 1 à 9, comprenant l'étape e) supplémentaire consistant à isoler la couche de sucre.

11. Procédé selon l'une quelconque des revendications 1 à 10, dans lequel le sucre est sélectionné dans le groupe consistant en l'acide glucuronique, le rhamnose, le fucose, le mannitol, le xylose, le mannose, le glucose et une combinaison de ceux-ci.

12. Procédé selon l'une quelconque des revendications 1 à 11, dans lequel la protéase est une protéase à sérine, une protéase à cystéine, une protéase à thréonine, une protéase aspartique, une protéase glutamique, une métalloprotéase et une asparagine peptide lyase ou une combinaison de celles-ci.

13. Utilisation d'un système d'enzyme comprenant une enzyme possédant une activité hydrolase telle que définie dans la revendication 1, dans un procédé selon l'une quelconque des revendications 1 à 12.
